# EUROPÄISCHE PATENTSCHRIFT

(11) **EP 2 358 469 B1**
(45) Veröffentlichungstag und Bekanntmachung des Hinweises auf die Patenterteilung: **24.10.2012**
(21) Anmeldenummer: 09759929.4
(22) Anmeldetag: 18.11.2009
(51) Int. Cl.: C10G 50/00, C07C 2/10

(54) **OLIGOMERISIERUNG VON OLEFINEN**
OLIGOMERISATION OF OLEFINS
OLIGOMÉRISATION D'OLÉFINES

(30) Priorität: 19.11.2008 EP 08169453
(43) Veröffentlichungstag der Anmeldung: 24.08.2011
(73) Patentinhaber: BASF SE, 67056 Ludwigshafen (DE)
(72) Erfinder: HEIDEMANN, Thomas, 68519 Viernheim (DE); ULONSKA, Armin, 67150 Niederkirchen (DE)
(86) Internationale Anmeldenummer: PCT/EP2009/065367
(87) Internationale Veröffentlichungsnummer: WO 2010/057905

(56) Entgegenhaltungen:
- DE-A1- 4 339 713
- DE-A1- 19 915 357
- DE-A1-102005 060 376
- JP-A- 2003 326 169
- US-A- 4 740 645
- US-A- 5 550 304

## Beschreibung

Die vorliegende Erfindung betrifft ein Verfahren zur Oligomerisierung von C₂- bis C₈-Olefinen an einem Nickel enthaltenden heterogenen Katalysator.

Olefine mit 2 bis 8 Kohlenstoffatomen oder deren Gemische stehen in großen Mengen sowohl aus FCC-Anlagen (Fluidized Catalyst Cracking) als auch aus Steamcrackern zur Verfügung. Es ist bekannt, die C₄-Fraktion, d.h. ein im Wesentlichen aus Butenen und Butanen bestehendes Gemisch, gegebenenfalls nach Abtrennung des iso-Butens, zur Herstellung von Oligomeren, insbesondere von Octenen und Dodecenen, zu verwenden. Sowohl die Octene als auch die Dodecene können nach der Hydroformylierung und anschließender Hydrierung zu den entsprechenden Alkoholen z.B. zur Herstellung von Weichmachern oder Tensiden verwendet werden.

Die Oligomerisierung wird großtechnisch entweder unter homogener oder heterogener Katalyse durchgeführt. Das homogen katalysierte Verfahren weist den Nachteil auf, dass der Katalysator vom Reaktionsgemisch getrennt werden muss. Dieser Abtrennungsschritt verursacht Abfallströme, die aufwendig aufgearbeitet werden müssen. Außerdem lässt sich der homogene Katalysator nicht regenerieren.

Die beschriebenen Nachteile bestehen bei der heterogen katalysierten Olefinoligomerisierung nicht. Die wichtigsten industriell ausgeübten heterogen katalysierten Olefinoligomerisierungsverfahren sind z.B. in A. Chauvel und G. Lefebvre, Petrochemical Process, Edition Technip (1989), S. 183-187 und F. Asinger, Die petrolchemische Industrie, Akademie-Verlag (1971), S. 278-299 angegeben.

Aus der DE-43 39 713 ist ein Verfahren zum Oligomerisieren von unverzweigten C₂-bis C₈-Olefinen an einem Festbettkatalysator bei erhöhtem Druck und erhöhter Temperatur bekannt, wobei der Katalysator als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumoxid enthält. Nach diesem Verfahren kann die Oligomerisierung von Butenen mit sehr guter Selektivität bezüglich linearer Produkte durchgeführt werden. Bevorzugte Verfahrensbedingungen sind in den WO 99/25668 und WO 00/53546 beschrieben.

Die DE-43 39 713 empfiehlt, den frisch hergestellten Katalysator vor dem Einsatz einer Konditionierung im trockenen Stickstoffstrom, z.B. bei Atmosphärendruck und Temperaturen von 200 bis 500 °C zu unterwerfen, um noch enthaltenes Wasser aus dem Katalysator zu entfernen. Die Druckschrift gibt keinen Restwassergehalt an, der nach der Konditionierung im Katalysator verbleibt. Es hat sich nun gezeigt, dass die Selektivität des Katalysators hinsichtlich der Bildung von Dimeren und Trimeren durch die Bedingungen der Konditionierung des Katalysators vor dem Kontakt mit dem Olefin beeinflusst werden kann.

Der vorliegenden Erfindung liegt die Aufgabe zugrunde, ein Verfahren zur Oligomerisierung von C₂- bis C₈-Olefinen anzugeben, bei dem die Selektivität hinsichtlich der Bildung höherer Oligomere, insbesondere Trimere, gegenüber der Bildung von Dimeren erhöht ist.

Demzufolge ist Gegenstand der Erfindung ein Verfahren zur Oligomerisierung von Olefinen durch Inkontaktbringen wenigstens eines C₂- bis C₈-Olefins mit einem Nickel enthaltenden heterogenen Katalysator, dadurch gekennzeichnet, dass man den Katalysator vor dem Kontakt mit dem Olefin durch Überleiten eines Inertgasstroms konditioniert, bis der abströmende Inertgasstrom einen Wassergehalt von weniger als 1000 ppm, vorzugsweise weniger als 500 ppm aufweist.

Da die direkte Bestimmung des Restwassergehalts im Katalysator während und nach der Konditionierung nur mit Schwierigkeiten möglich ist, dient in der vorliegenden Erfindung der Wassergehalt des abströmenden Inertgasstroms als Maß für den Fortschritt der Konditionierung. Diese Vorgehensweise gestattet eine einfache Kontrolle und Steuerung der Konditionierungsbedingungen.

Das erfindungsgemäße Verfahren gestattet die Herstellung von Olefinoligomeren, wobei das Gewichtsverhältnis der gebildeten Olefintrimeren und höheren Oligomeren zu den Olefindimeren z. B. mehr als 0,2 beträgt.

Die verwendbaren heterogenen Nickel enthaltenden Katalysatoren können unterschiedliche Struktur aufweise. Es kommen an sich bekannte Katalysatoren in Betracht, wie sie in C.T. O'Connor et al., Catalysis Today, Bd.6 (1990), S.336-338 beschrieben sind. Insbesondere werden trägergebundene Nickelkatalysatoren eingesetzt. Die Trägermaterialien können z.B. Kieselsäure, Tonerde, Aluminosilicate, Aluminosilicate mit Schichtstrukturen und Zeolithe, wie Mordenit, Faujasit, Zeolith X, Zeolith-Y und ZSM-5, Zirkoniumoxid, das mit Säuren behandelt ist, oder sulfatiertes Titandioxid sein. Besonders geeignet sind Fällungskatalysatoren, die durch Mischen wässriger Lösungen von Nickelsalzen und Silicaten, z.B. Natriumsilicat mit Nickelnitrat, und gegebenenfalls Aluminiumsalzen, wie Aluminiumnitrat, und Calcinieren erhältlich sind. Weiterhin sind Katalysatoren verwendbar, die durch Einlagerung von Ni²⁺-Ionen durch Ionenaustausch in natürliche oder synthetische Schichtsilicate, wie Montmorillonite, erhalten werden. Geeignete Katalysatoren können auch durch Imprägnieren von Kieselsäure, Tonerde oder Alumosilicaten mit wässrigen Lösungen löslicher Nickelsalze, wie Nickelnitrat, Nickelsulfat oder Nickelchlorid, und anschließende Calcinierung erhalten werden.

Nickeloxid enthaltende Katalysatoren sind bevorzugt. Besonders bevorzugt sind Katalysatoren, die im Wesentlichen aus NiO, SiO₂, TiO₂ und/oder ZrO₂ sowie gegebenenfalls Al₂O₃ bestehen. Derartige Katalysatoren sind insbesondere bevorzugt, wenn das erfindungsgemäße Verfahren zur Oligomerisierung von Butenen oder Pentenen herangezogen wird. Sie liefern überwiegend lineare Produkte. Am meisten bevorzugt ist ein Katalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält. Ein solcher Katalysator ist durch Fällung der Katalysatormasse bei pH 5 bis 9 durch Zugabe einer Nickelnitrat enthaltenden wässrigen Lösung zu einer Alkaliwasserglaslösung, die Titandioxid und/oder Zirkondioxid enthält, Filtrieren, Trocknen und Tempern bei 350 bis 650 °C erhältlich. Zur Herstellung dieser Katalysatoren wird im Einzelnen auf die DE-43 39 713 verwiesen. Auf die Offenbarung dieser Druckschrift und den darin zitierten Stand der Technik wird vollinhaltlich Bezug genommen.

Der Katalysator liegt vorzugsweise in stückiger Form, z.B. in Form von Tabletten, z.B. mit einem Durchmesser von 2 bis 6 mm und einer Höhe von 3 bis 5 mm, Ringen mit z.B. 5 bis 7 mm Außendurchmesser, 2 bis 5 mm Höhe und 2 bis 3 mm Lochdurchmesser, oder Strängen unterschiedlicher Länge eines Durchmessers von z.B. 1,5 bis 5 mm, vor. Derartige Formen werden auf an sich bekannte Weise durch Tablettierung oder Extrusion, meist unter Verwendung eines Tablettierhilfsmittels, wie Graphit oder Stearinsäure, erhalten.

Zur Konditionierung wird der Katalysator in Gegenwart eines Inertgases auf eine Temperatur von mehr als 100 °C, vorzugsweise 100 bis 500 °C, z. B. 150 bis 500 °C, erwärmt. Das Erwärmen kann einige Stunden bis mehrere Tage, z.B. 6 bis 150 Stunden, vorzugsweise 30 bis 72 Stunden, dauern. Vorzugsweise wird eine Katalysatorschüttung vom Inertgas, das unter Normaldruck oder erhöhtem Druck, z. B. bei einem Druck von 0,9 bis 1,5 bar, vorliegen kann, durchströmt.

Zum Erwärmen der Katalysatorschüttung ist beispielsweise eine im Reaktormantel vorgesehen Heizeinrichtung geeignet, wenn die Konditionierung in situ im Oligomerisierungsreaktor durchgeführt wird.

Als Inertgas sind Gase geeignet, die am Katalysator bei der Konditionierungstemperatur im Wesentlichen keine chemische Umwandlung erfahren und mit dem Katalysator im Wesentlichen keine chemische oder physikalische Wechselwirkung eingehen. Geeignet sind z.B. Stickstoff, Argon oder Neon, wovon Stickstoff am meisten bevorzugt ist. Im Allgemeinen weist der zuströmende Inertgasstrom einen Wassergehalt von weniger als 250 ppm, insbesondere weniger als 100 ppm auf.

In bevorzugten Ausführungsformen leitet man 10 bis 1000 Nl/h, insbesondere 50 bis 500 Nl/h Inertgas pro l Katalysatorschüttung über den Katalysator. Unter Normliter (NI) wird eine Gasmenge verstanden, die unter Normbedingungen (20 °C, 1013 hPa) ein Volumen von 1 l einnimmt.

Erfindungsgemäß wird der Wassergehalt im abströmenden Inertgasstrom gemessen; die Messung kann periodisch oder kontinuierlich erfolgen. Zur Bestimmung des Wassergehalts im abströmenden Inertgasstrom ist beispielsweise ein kapazitiver Feuchtesensor, z. B. der Feuchtesensor Minicap 2 der Firma GE Parametrics, geeignet. Vor der Messung kann der Inertgasstrom auf eine geeignete Messtemperatur gebracht werden, z. B. etwa 60 °C.

Nachdem ein angestrebter Wassergehalt im abströmenden Inertgasstrom erreicht ist, lässt man die Katalysatorschüttung vorzugsweise im Inertgasstrom abkühlen, z. B. auf unter 40 °C.

Die Konditionierung und die Oligomerisierung können räumlich und/oder zeitlich getrennt voneinander durchgeführt werden. Dieser Fall liegt beispielsweise regelmäßig vor, wenn die Konditionierung im zeitlichen und/oder räumlichen Zusammenhang mit der Katalysatorherstellung erfolgt. Im Allgemeinen ist es jedoch bevorzugt, dass die Konditionierung und die Oligomerisierung im gleichen Reaktor durchgeführt werden. Es ist weiter bevorzugt, die Konditionierung des Katalysators unmittelbar vor Beginn der Oligomerisierung durchzuführen.

Vor der eigentlichen Oligomerisierung kann der Katalysator einer Vorbehandlung unterzogen werden, bei der dieser mit einem gegenüber dem eigentlichen Produkteinsatzgemisch olefinärmeren Kohlenwasserstoffgemisch in Kontakt gebracht wird, wie in der WO 00/059849 beschrieben. Der Kontakt des Katalysators mit Kohlenwasserstoffen ist in der Regel von einem Temperaturanstieg aufgrund der freiwerdenden Adsorptionsenthalpie begleitet. Findet dieser Vorgang gleichzeitig mit der exothermen Oligomerisierungsreaktion statt, kann der Katalysator unter Umständen zu hohen Temperaturen ausgesetzt werden, die die Aktivität und Lebensdauer des Katalysators beeinträchtigen. Wird der Katalysator aber zuerst mit einem olefinarmen Kohlenwasserstoffgemisch in Kontakt gebracht, in dem oligomerisierungsfähige Olefine kaum oder nicht vorhanden sind, kann die Adsorptionswärme problemlos abgeführt werden.

Zur Oligomerisierung wird der Katalysator mit wenigstens einem C₂- bis C₈-Olefin in Kontakt gebracht, wie Ethylen, Propylen, n-Buten, n-Penten und n-Hexen. Vorzugsweise umfasst das n-Buten und/oder n-Penten.

Bei den großtechnisch zugänglichen Olefin-haltigen, zur Durchführung des erfindungsgemäßen Verfahrens geeigneten Kohlenwasserstoffströmen handelt es in der Regel um Gemische. Es eignen sich Kohlenwasserstoffgemische eines Gehalts an C₂- bis C₈-Olefinen von 50 bis 100 Gew.-%, vorzugsweise 60 bis 100 Gew.-%. Im Allgemeinen handelt es sich beim Olefinanteil im Wesentlichen um ein Olefin, wie Propylen oder ein Gemisch von Olefinen gleicher Anzahl von C-Atomen, wie isomere Butene. Das Kohlenwasserstoffgemisch kann neben C₂- bis C₈-Olefinen einen der Oligomerisierung nicht zugänglichen Inertanteil enthalten. Dieser Inertanteil kann z. B. aus gesättigten Kohlenwasserstoffen, wie Alkanen, z.B. Propan, Butan, Pentan, Hexan, Heptan, Octan, Nonan, Decan, Undecan und/oder Dodekan, und/oder Cycloalkanen bestehen. In der Regel weisen die gesättigten Kohlenwasserstoffe die gleiche Anzahl an C-Atomen oder eine um ein C-Atom höhere oder niedrigere Anzahl an C-Atomen auf wie das Olefin.

Ein bevorzugtes Kohlenwasserstoffgemisch enthält 50 bis 100 Gew.-%, vorzugsweise 60 bis 90 Gew.-%, Butene und 0 bis 50 Gew.-%, vorzugsweise 10 bis 40 Gew.-%, Butane. Vorzugsweise umfasst die Butenfraktion weniger als 5 Gew.-%, insbesondere weniger als 3 Gew.-% iso-Buten (bezogen auf die Butenfraktion). Die Butenfraktion weist im Allgemeinen folgende Zusammensetzung auf (jeweils bezogen auf die Butenfraktion):

| | |
|---|---|
| 1-Buten | 1 bis 50 Gew.-%, |
| cis-2-Buten | 1 bis 50 Gew.-%, |
| trans-2-Buten | 1 bis 99 Gew.-%, |
| iso-Buten | 1 bis 5 Gew.-%. |

Als besonders bevorzugter Einsatzstoff wird das sogenannte Raffinat II verwendet, bei dem es sich um einen iso-Buten-abgereicherten C₄-Schnitt aus einer FCC-Anlage oder einem Steamcracker handelt. Raffinat II weist folgende typische Zusammensetzung auf:

| | |
|---|---|
| i-,n-Butan | 26 Gew.-%; |
| i-Buten | 1 Gew.-% |
| 1-Buten | 26 Gew.-%, |
| trans-2-Buten | 31 Gew.-%, |
| cis-2-Buten | 16 Gew.-%. |

Sind Diolefine oder Alkine im Kohlenwasserstoffgemisch vorhanden, so werden diese vor der Oligomerisierung vorzugsweise auf weniger als 10 Gew.-ppm, bevorzugt weniger als 5 Gew.-ppm, insbesondere weniger als 1 Gew.-ppm, aus demselben entfernt. Sie werden bevorzugt durch selektive Hydrierung, z.B. gemäß EP-81041 und DE-1568542 entfernt.

Die großtechnisch zugänglichen Kohlenwasserstoffgemische, die als Kohlenwasserstoffgemisch für die Zwecke der vorliegenden Erfindung geeignet sind, enthalten oftmals Verbindungen, die als Katalysatorgifte wirken und den Oligomerisierungskatalysator deaktivieren. Hierzu zählen sauerstoffhaltige Verbindungen, wie Alkohole, Aldehyde, Ketone und Ether, sowie stickstoffhaltige, schwefelhaltige und halogenhaltige Verbindungen. Die Anwesenheit solcher Katalysatorgifte würde zu einer unerwünschten Verringerung der Katalysatoraktivität führen. Aus dem Kohlenwasserstoffgemisch werden zweckmäßigerweise sauerstoffhaltige Verbindungen, wie Alkohole, Aldehyde, Ketone oder Ether, weitgehend entfernt. Die Konzentration an sauerstoffhaltigen, schwefelhaltigen, stickstoffhaltigen und halogenhaltigen Verbindungen im Kohlenwasserstoffgemisch beträgt vorzugsweise weniger als 1 Gew.-ppm, insbesondere weniger als 0,5 Gew.-ppm.

Gemäß einem bevorzugten Aspekt der Erfindung wird das Kohlenwasserstoffgemisch daher vor dem Inkontaktbringen mit dem Katalysator zur Entfernung von Katalysatorgiften über ein Adsorptionsmittel geleitet. Als Adsorptionsmittel sind Molekularsiebe, vorzugsweise mit einem Porendurchmesser von größer als 4 Å bis 15 Å, geeignet. Als Molekularsiebe können kristalline, natürliche Aluminiumsilicate, wie z.B. Schichtgittersilicate, wie auch synthetische Molekularsiebe eingesetzt werden. Weiter sind kommerzielle Molekularsiebe, wie z.B. Typen der Fa. Bayer AG, Dow, Union Carbide, Laporte oder Mobil, geeignet. Diese Molekularsiebe können z.B. Zeolithe vom A-, X- und Y-Typ sein. Ferner sind auch synthetische Molekularsiebe geeignet, die neben Silicium und Aluminium als Hauptbestandteile noch andere Atome als Nebenbestandteile aufweisen. Diese können z.B. durch einen Ionenaustausch mit den austauschbaren Kationen in den Zeolithen eingebaut werden. Beispielhaft sei hier der Austausch mit seltenen Erden, wie z.B. Gallium, Indium oder Lanthan, oder mit Nickel, Cobalt, Kupfer, Zink oder Silber, aufgeführt.

Darüber hinaus können auch synthetische Zeolithe, in welchen andere Atome, wie z.B. Bor oder Phosphor, die durch Kopräzipitation in das Gitter mit eingebaut sind, eingesetzt werden.

Weitere geeignete Adsorptionsmittel sind z.B. Aluminiumoxide, Aluminiumphosphate, Siliciumdioxide, Kieselgur, Titandioxide, Zirkondioxide, polymere Adsorbentien und Gemische davon. Das Überleiten des Kohlenwasserstoffgemisches über das Adsorptionsmittel erfolgt zweckmäßigerweise in einem Festbett oder einem Wanderbett. Das Kohlenwasserstoffgemisch kann beim Überleiten über das Adsorptionsmittel in gasförmiger oder flüssiger Phase vorliegen, liegt aber vorzugsweise in flüssiger Phase vor.

Die Oligomerisierung erfolgt vorzugsweise bei Temperaturen von 20 bis 300 °C, vorzugsweise 30 bis 280 °C, insbesondere 30 bis 140 °C und besonders bevorzugt von 40 bis 130 °C. Sie erfolgt vorzugsweise bei einem Druck von 1 bis 100 bar, vorzugsweise 10 bis 300 bar, insbesondere von 15 bis 100 bar und besonders bevorzugt von 20 bis 80 bar. Der Druck wird dabei zweckmäßigerweise so eingestellt, dass bei der gewählten Temperatur das Kohlenwasserstoffgemisch flüssig oder im überkritischen Zustand vorliegt.

Geeignete gegebenenfalls druckfeste Reaktionsapparaturen für das Inkontaktbringen des Kohlenwasserstoffgemisches mit dem heterogenen Katalysator sind dem Fachmann bekannt. Dazu zählen die allgemein üblichen Reaktoren für Gas/Fest-Reaktionen bzw. Flüssig/Fest-Reaktionen. Geeignet sind z.B. Rohrbündelreaktoren oder Schachtöfen. Aufgrund der geringeren Investitionskosten sind Schachtöfen bevorzugt. Das erfindungsgemäße Oligomerisierungsverfahren kann in einem einzelnen Reaktor durchgeführt werden, wobei der Oligomerisierungskatalysator in einem einzigen oder mehreren Festbetten im Reaktor angeordnet sein kann. Alternativ kann zur Durchführung des erfindungsgemäßen Verfahrens eine Reaktorkaskade aus mehreren, vorzugsweise zwei, hintereinandergeschalteten Reaktoren eingesetzt werden, wobei beim Passieren des bzw. der dem letzten Reaktor der Kaskade vorgeschalteten Reaktors bzw. Reaktoren die Oligomerisierung der Olefine im Reaktionsgemisch nur bis zu einem Teilumsatz betrieben wird und der gewünschte Endumsatz erst beim Passieren des Reaktionsgemisches durch den letzten Reaktor der Kaskade erzielt wird. In den einzelnen Reaktoren der Reaktorkaskade kann der Oligomerisierungskatalysator in einem einzigen oder in mehreren Katalysatorbetten angeordnet sein. Weiterhin können in den einzelnen Reaktoren der Reaktorkaskade unterschiedliche Reaktionsbedingungen hinsichtlich Druck und/oder Temperatur im Rahmen der oben genannten Druck- und Temperaturbereiche eingestellt werden. Außerdem ist es möglich, in den einzelnen Reaktoren der Kaskade unterschiedliche Oligomerisierungskatalysatoren einzusetzen, obgleich die Anwendung des gleichen Katalysators in sämtlichen Reaktoren der Kaskade bevorzugt ist. Bei dem bevorzugten Reaktor handelt es sich in der Regel um ein mit dem Katalysator beschicktes, vertikales zylindrisches Rohr, das von dem Kohlenwasserstoffgemisch z.B. von oben nach unten durchströmt wird.

Nach dem Verlassen des Reaktors bzw. des letzten Reaktors einer Kaskade werden aus dem Reaktoraustrag die gebildeten Oligomere von den nicht umgesetzten Olefinen und gesättigten Kohlenwasserstoffen abgetrennt. Die gebildeten Oligomere können in einem nachfolgenden Vakuumfraktionierungsschritt aufgereinigt werden.

In einer bevorzugten Ausführungsform wird der von den gebildeten Oligomeren befreite Reaktoraustrag, der im Wesentlichen aus nicht umgesetzten Olefinen und gesättigten Kohlenwasserstoffen besteht, vollständig oder zum Teil zurückgeführt. Es ist bevorzugt, das Rückführverhältnis so zu wählen, dass die Konzentration an Oligomeren im Reaktionsgemisch an keiner Stelle des Reaktors (bzw. der Reaktorkaskade) 35 Gew.-%, vorzugsweise 20 Gew.-%, bezogen auf das Reaktions-Kohlenwasserstoffgemisch, übersteigt.

Die Betriebsphase kann mit Vorteil adiabatisch durchgeführt werden. Die Oligomerisierungsreaktion verläuft in der Regel exotherm. Das Reaktionsgemisch erfährt daher beim Durchströmen des Katalysatorbettes eine Temperaturerhöhung. Unter adiabatischer Reaktionsführung wird im Unterschied zur isothermen Reaktionsführung, bei der die in einer exothermen Reaktion entstehende Wärmemenge durch Kühlung mittels Kühl- oder Thermostatiervorrichtungen abgeführt wird und so die Temperatur im Reaktor konstant, d.h. isotherm, gehalten wird, eine Betriebsweise verstanden, bei der die in einer exothermen Reaktion freiwerdende Wärmemenge von der Reaktionsmischung im Reaktor aufgenommen und keine Kühlung durch Kühlvorrichtungen angewandt wird. Es versteht sich, dass ein vernachlässigbar kleiner Teil der bei der exothermen Reaktion freiwerdenden Wärmemenge unvermeidlich auch vom Reaktorkörper aufgenommen und durch Wärmeleitung und -abstrahlung an die Umwelt abgegeben wird. Im technischen Sinne wird deshalb unter einer adiabatischen Reaktionsführung oder Betriebsweise eine Reaktionsführung oder Betriebsweise verstanden, bei der, abgesehen von dem durch natürliche Wärmeleitung und -abstrahlung vom Reaktor an die Umgebung abgegebenen Teil der Reaktionswärme, die gesamte Reaktionswärme vom Reaktionsgemisch aufgenommen und mit diesem aus dem Reaktor abgeführt wird. Es bestehen zwei grundsätzliche Möglichkeiten zur Steuerung der Reaktionstemperatur. Da die exotherme Reaktion im Fall des vorliegenden Oligomerisierungsverfahrens durch den Kontakt der Olefine mit dem Katalysator zustande kommt und somit nur im Bereich der Katalysatorschüttung Wärme freigesetzt wird, kann die Temperatur im Reaktor durch Einstellen der Konzentration an Olefin im eintretenden Kohlenwasserstoffgemisch gesteuert werden. Diese wird ihrerseits zweckmäßigerweise durch die Rückführung der vom oligomeren Produkt abgetrennten, nicht umgesetzten Olefine und gesättigten Kohlenwasserstoffe zurück in den Oligomerisierungsreaktor geregelt werden. Da der in den Oligomerisierungsreaktor zurückgeführte Strom einen geringeren Gehalt an reaktiven Olefinen und einen höheren Gehalt an unter den Reaktionsbedingungen inerten gesättigten Kohlenwasserstoffe hat als der frisch zugeführte Einsatzkohlenwasserstoffstrom, bewirkt der diesem Strom zugemischte Rückführstrom eine Verdünnung des Olefingehaltes. Über das Verhältnis von Rückführstrom zu frischen Einsatzkohlenwasserstoffstrom kann daher mittelbar die Reaktortemperatur gesteuert werden.

Eine weitere Möglichkeit der Verfahrensteuerung besteht in der Regelung der Eintrittstemperatur des Kohlenwasserstoffgemisches. Eine tiefere Temperatur des eintretenden Kohlenwasserstoffgemischs führt zu einer verbesserten Abführung der Reaktionswärme. Andererseits kann beim Nachlassen der Katalysatoraktivität die Eintrittstemperatur des Kohlenwasserstoffgemisches höher gewählt werden, um eine höhere Reaktionsgeschwindigkeit zu erreichen und damit die nachlassende Katalysatoraktivität zu kompensieren. Die Eintrittstemperatur des Kohlenwasserstoffgemischs ist in der Regel durch Sicherheitsaspekte und praktische Erwägungen begrenzt. Die maximale Eintrittstemperatur liegt für ein überwiegend Butene und gegebenenfalls Butane enthaltendes Kohlenwasserstoffgemisch im Allgemeinen bei 130 °C. Ist die maximale Eintrittstemperatur des Kohlenwasserstoffgemisches erreicht, ist der Katalysator erschöpft und muss durch neuen Katalysator ersetzt werden. Der erschöpfte Katalysator kann gegebenenfalls regeneriert werden.

Die erfindungsgemäße Oligomerisierung wird vorzugsweise so gesteuert, dass die Temperaturtönung über ein Katalysatorbett nicht mehr als 50 °C, insbesondere nicht mehr als 40 °C, besonders bevorzugt nicht mehr als 30 °C beträgt. Als Temperaturtönung wird die Differenz zwischen der Eintrittstemperatur des Kohlenwasserstoffgemischs und der Austrittstemperatur des Reaktionsgemischs betrachtet. Die adiabatische Betriebsweise umfasst auch eine Verfahrenskonfiguration des erfindungsgemäßen Verfahrens, bei der die Umsetzung der Olefine zu Oligomeren auf eine Reaktorkaskade aus zwei oder mehreren, vorzugsweise zwei, Oligomerisierungsreaktoren verteilt wird und das teilumgesetzte Reaktionsgemisch nach Verlassen des einen Reaktors und vor Eintritt in den nachfolgenden Reaktor der Kaskade mittels herkömmlicher Kühlvorrichtungen, wie Kühlmäntel oder Wärmetauscher, gekühlt wird. Bei geeigneter Fahrweise wird pro Katalysatorbett ein Umsatz, bezogen auf den Olefinanteil des Kohlenwasserstoffgemischs, von 15 bis 50 Gew.-% erreicht.

Die Erfindung wird durch die folgenden Beispiele näher veranschaulicht.

### BEISPIELE

Als Katalysator in den nachstehenden Beispielen 1 und 2 dienten 100 l eines Materials, das gemäß DE 43 39 713 zu 3*3 mm Volltabletten geformt wurde (Zusammensetzung in Gew.-%: 50 % NiO, 12,5 % TiO₂, 33,5 % SiO₂, 4 % Al₂O₃).

In den Beispielen wurde ein Raffinat II mit folgender mittlerer Zusammensetzung eingesetzt:

| | |
|---|---|
| i-Butan: | 5 Gew.-% |
| n-Butan: | 18 Gew.-% |
| i-Buten: | 2 Gew.-% |
| Buten-1: | 31 Gew.-% |
| Buten-2-trans: | 28 Gew.-% |
| Buten-2-cis: | 16 Gew.-% |

Die Versuche wurden in einer Reaktorkaskade, bestehend aus zwei hintereinandergeschalteten Reaktoren (Durchmesser 80 mm, Länge 4000 mm, Zwischenkühlung zwischen den beiden Reaktoren) mit anschließender Destillationskolonne durchgeführt. Zum Reaktoreingang des ersten Reaktors wurde unter Reaktionsbedingungen ein Gemisch aus Raffinat II gemäß obiger Zusammensetzung und ein Rückführstrom vom Kopf der Destillationskolonne (nach Abrennung der C₈₊-Kohlenwasserstoffe) geleitet.

Jeweils 20 l Katalysator wurde in beide Reaktoren eingefüllt und unter Durchleiten von 100 Nl/l*h trockenem N₂ (60 ppm Feuchtegehalt) bei Normaldruck sowie einer Reaktortemperatur von 190 °C getrocknet. Der Trocknungsverlauf wurde mit Hilfe einer Feuchtemessung (Parametrics) des den Reaktor verlassenden Gasstroms nach Abkühlung auf 60 °C verfolgt. Nach Erreichen der gewünschten Restfeuchte wurde der Reaktor abgekühlt und die Konditionierung im N₂-Strom beendet. Anschließend wurden Raffinat-II- und Rückführstrom-Menge (Summe aus beiden konstant 50 kg/h) sowie Druck und Temperatur (mittlere Temperatur ist definiert als der Mittelwert aus den jeweiligen Reaktoreingangs- und Reaktorausgangstemperaturen) gemäß der nachfolgenden Tabelle eingestellt. Erfindungsgemäßes Beispiel und Vergleichsbeispiel wurden jeweils mit neuem Katalysator aus der einheitlichen 100-I-Katalysatorcharge durchgeführt.

Die Tabelle 1 gibt die erhaltenen Ergebnisse in Abhängigkeit der Konditionierbedingungen wieder:

**Tabelle 1: Oligomerisierungsbedingungen und -ergebnisse**

| Beispiel 1: erfindungsgemäß | | | | | | | | | | | |
|---|---|---|---|---|---|---|---|---|---|---|---|
| Konditionierung | Raffinat II | Rückführstrom | Mittlere Reaktionstemperatur | Druck | C₄-Umsatz | C₈₊-Konz. am Reaktorausgang | C₈-Sel. | C₈-Menge | C₁₂-Sel. | C₁₂-Menge | C₁₂/C₈ |
| 36 h Trocknung mit 100 Nl/l*h N₂, Restfeuchte 400 ppm | kg/h | kg/h | °C | bar | % | % | % | kg/h | % | kg/h | kg/kg |
| E1 | 17 | 33 | 60 | 30 | 77 | 19% | 77 | 7,8 | 17 | 1,7 | 0,22 |

| Beispiel 2: Vergleich | | | | | | | | | | | |
|---|---|---|---|---|---|---|---|---|---|---|---|
| Konditionierung | Raffinat II | Rückführstrom | Mittlere Reaktionstemperatur | Druck | C₄-Umsatz | C₈₊-Konz. am Reaktorausgang | C₈-Sel. | C₈-Menge | C₁₂-Sel. | C₁₂-Menge | C₁₂/C₈ |
| 24 h Trocknung mit 100 Nl/l*h N₂, Restfeuchte 1600 ppm | kg/h | kg/h | ° C | bar | % | % | % | kg/h | % | kg/h | kg/kg |
| V1 | 17 | 33 | 58 | 30 | 68 | 17 | 81 | 7,2 | 14 | 1,2 | 0,17 |
| V2 | 14 | 36 | 56 | 30 | 76 | 15 | 81 | 6,6 | 14 | 1,1 | 0,17 |
| V3 | 20 | 30 | 61 | 30 | 65 | 19 | 81 | 8,1 | 14 | 1,4 | 0,17 |

In der Tabelle 1 ist die C₈- bzw. C₁₂-Selektivität definiert als die Menge der gebildeten Octene bzw. Dodecene, bezogen auf die umgesetzte Menge Butene.

Im erfindungsgemäßen Beispiel E1 wurde bei einem Butenumsatz von 77 % und einer daraus resultierenden C₈₊-Konzentration von 19 % am Reaktorausgang eine C₈-Selektivität von 77 % und eine C₁₂-Selektivität von 17 % erreicht. Die produzierte C₁₂-Menge liegt bei 0,22 kg pro kg C₈.

Im Vergleichsbeispiel V1 wurden analoge Bedingungen zu E1 eingestellt. Dies führte gegenüber E1 zu leicht verringerten Buten-Umsätzen und C₈₊-Konzentrationen am Reaktorausgang. Daher wurden zusätzlich die Beispiele V2 und V3 durchgeführt, in denen die zugeführte Menge Raffinat II verringert bzw. erhöht wurde, um einen mit E1 vergleichbaren Buten-Umsatz bzw. eine mit E1 vergleichbare C₈₊-Konzentration am Reaktorausgang einzustellen. Im Vergleich zum Beispiel E1 wird bei allen drei Vergleichsbeispielen eine C₈-Selektivität von 81 % und eine C₁₂-Selektivität von 14 % erreicht. Die produzierte C₁₂-Menge liegt bei 0,17 kg pro kg C₈.

Die Beispiele verdeutlichen, dass eine Trocknung bis auf eine Restfeuchte < 1000 ppm zu einer Erhöhung der C₁₂-Selektivität um 3 %-Punkte führt, was bezogen auf eine gegebene Menge C₈ einer C₁₂-Kapazitätserhöhung um 30 % entspricht. Zusätzlich ist auch noch eine Aktivitätserhöhung zu beobachten.

## Patentansprüche

1. Verfahren zur Oligomerisierung von Olefinen durch Inkontaktbringen wenigstens eines C₂- bis C₈-Olefins mit einem Nickel enthaltenden heterogenen Katalysator, **dadurch gekennzeichnet, dass** man den Katalysator vor dem Kontakt mit dem Olefin durch Überleiten eines Inertgasstroms konditioniert, bis der abströmende Inertgasstrom einen Wassergehalt von weniger als 1000 ppm aufweist.

2. Verfahren nach Anspruch 1, **dadurch gekennzeichnet, dass** der abströmende Inertgasstrom einen Wassergehalt von weniger als 500 ppm aufweist.

3. Verfahren nach Anspruch 1 oder 2, **dadurch gekennzeichnet, dass** das Inertgas Stickstoff ist.

4. Verfahren nach einem der vorhergehenden Ansprüche, **dadurch gekennzeichnet, dass** der zuströmende Inertgasstrom einen Wassergehalt von weniger als 100 ppm aufweist.

5. Verfahren nach einem der vorhergehenden Ansprüche, **dadurch gekennzeichnet, dass** man den Katalysator bei einer Temperatur von 100 bis 500 °C konditioniert.

6. Verfahren nach einem der vorhergehenden Ansprüche, **dadurch gekennzeichnet, dass** man den Katalysator bei einem Druck von 0,9 bis 1,5 bar konditioniert.

7. Verfahren nach einem der vorhergehenden Ansprüche, **dadurch gekennzeichnet, dass** man 50 bis 500 Nl/h Inertgas pro l Katalysatorschüttung über den Katalysator leitet.

8. Verfahren nach einem der vorhergehenden Ansprüche, **dadurch gekennzeichnet, dass** das Olefin n-Buten und/oder n-Penten umfasst.

9. Verfahren nach einem der vorhergehenden Ansprüche, **dadurch gekennzeichnet, dass** man das Olefin bei einer Temperatur von 20 bis 300 °C und einem Druck von 1 bis 100 bar mit dem Katalysator in Kontakt bringt.

10. Verfahren nach einem der vorhergehenden Ansprüche, **dadurch gekennzeichnet, dass** der Katalysator im Wesentlichen aus NiO, SiO₂, TiO₂ und/oder ZrO₂ sowie gegebenenfalls Al₂O₃ besteht.

11. Verfahren nach Anspruch 10, **dadurch gekennzeichnet, dass** der Katalysator als als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält.

12. Verfahren nach einem der vorhergehenden Ansprüche, **dadurch gekennzeichnet, dass** das Gewichtsverhältnis der gebildeten Olefintrimeren und höheren Oligomeren zu den Olefindimeren mehr als 0,2 beträgt.

## Claims

1. A process for oligomerizing olefins by bringing at least one C₂-C₈-olefin into contact with a nickel-comprising heterogeneous catalyst, wherein the catalyst is conditioned by passing an inert gas stream over it until the outflowing inert gas stream has a water content of less than 1000 ppm before contact with the olefin.

2. The process according to claim 1, wherein the outflowing inert gas stream has a water content of less than 500 ppm.

3. The process according to claim 1 or 2, wherein the inert gas is nitrogen.

4. The process according to any of the preceding claims, wherein the inflowing inert gas stream has a water content of less than 100 ppm.

5. The process according to any of the preceding claims, wherein the catalyst is conditioned at a temperature of from 100 to 500°C.

6. The process according to any of the preceding claims, wherein the catalyst is conditioned at a pressure of from 0.9 to 1.5 bar.

7. The process according to any of the preceding claims, wherein from 50 to 500 standard l/h of inert gas per l of catalyst bed are passed over the catalyst.

8. The process according to any of the preceding claims, wherein the olefin comprises n-butene and/or n-pentene.

9. The process according to any of the preceding claim, wherein the olefin is brought into contact with the catalyst at a temperature of from 20 to 300°C and a pressure of from 1 to 100 bar.

10. The process according to any of the preceding claims, wherein the catalyst comprises essentially NiO, SiO₂, TiO₂ and/or ZrO₂ and optionally Al₂O₃.

11. The process according to claim 10, wherein the catalyst comprises, as significant active constituents, from 10 to 70% by weight of nickel oxide, from 5 to 30% by weight of titanium dioxide and/or zirconium dioxide, from 0 to 20% by weight of aluminum oxide and silicon dioxide as balance.

12. The process according to any of the preceding claims, wherein the weight ratio of the olefin trimers and higher oligomers formed to the olefin dimers is greater than 0.2.

## Revendications

1. Procédé pour l'oligomérisation d'oléfines par mise en contact d'au moins une C₂-C₈-oléfine avec un catalyseur hétérogène contenant du nickel, **caractérisé en ce qu'**on conditionne le catalyseur avant 1a mise en contact avec l'oléfine en faisant passer un flux de gaz inerte, jusqu'à ce que le flux de gaz inerte qui s'écoule du catalyseur présente une teneur en eau inférieure à 1000 ppm.

2. Procédé selon la revendication 1, **caractérisé en ce que** le flux de gaz inerte qui s'écoule du catalyseur présente une teneur en eau inférieure à 500 ppm.

3. Procédé selon la revendication 1 ou 2, **caractérisé en ce que** le gaz inerte est de l'azote.

4. Procédé selon l'une quelconque des revendications précédentes, **caractérisé en ce que** le flux de gaz inerte qui s'écoule vers le catalyseur présente une teneur en eau inférieure à 100 ppm.

5. Procédé selon l'une quelconque des revendications précédentes, **caractérisé en ce qu'**on conditionne le catalyseur à une température de 100 à 500°C.

6. Procédé selon l'une quelconque des revendications précédentes, **caractérisé en ce qu'**on conditionne le catalyseur à une pression de 0,9 à 1,5 bar.

7. Procédé selon l'une quelconque des revendications précédentes, **caractérisé en ce qu'**on fait passer 50 à 500 Nl/h de gaz inerte, par l de catalyseur en vrac, sur le catalyseur.

8. Procédé selon l'une quelconque des revendications précédentes, **caractérisé en ce que** l'oléfine est composée de n-butène et/ou de n-pentène.

9. Procédé selon l'une quelconque des revendications précédentes, **caractérisé en ce qu'**on met en contact l'oléfine avec le catalyseur à une température de 20 à 300°C et à une pression de 1 à 100 bars.

10. Procédé selon l'une quelconque des revendications précédentes, **caractérisé en ce que** le catalyseur est essentiellement constitué de NiO, de SiO₂, de TiO₂ et/ou de ZrO₂ ainsi que le cas échéant d'Al₂O₃.

11. Procédé selon la revendication 10, **caractérisé en ce que** le catalyseur contient, comme constituants actifs essentiels, 10 à 70% en poids d'oxyde de nickel, 5 à 30% en poids de dioxyde de titane et/ou de dioxyde de zirconium, 0 à 20% en poids d'oxyde d'aluminium et pour le reste du dioxyde de zirconium.

12. Procédé selon l'une quelconque des revendications précédentes, **caractérisé en ce que** le rapport pondéral des trimères d'oléfine et des oligomères supérieurs formés aux dimères d'oléfine est supérieur à 0,2.
